# EUROPEAN PATENT APPLICATION

(11) **EP 4 505 945 A1**
(43) Date of publication of application: **12.02.2025**
(21) Application number: 24186838.9
(22) Date of filing: 05.07.2024
(51) Int. Cl.: A61B 8/00, A61B 5/00

(54) **TRANSPARENT ULTRASOUND PROBE MODULE AND OPTICAL APPARAT US HAVING THE SAME**

(30) Priority: 08.08.2023 KR 20230103805
(71) Applicant: Postech Research And Business Development Foundation (Postech), Gyeonggi-do 37673 (KR)
(72) Inventor: PARK, Jeong Woo, 37673 Gyeongsangbuk do (KR); KIM, Chul Hong, 37673 Gyeongsangbuk do (KR); KIM, Hyung Ham, 37673 Gyeongsangbuk do (KR); YOO, Hyung Ham, 37673 Gyeongsangbuk do (KR)
(74) Representative: AWA Sweden AB

(57) **Abstract**

An ultrasound probe module provided inside a housing of an optical apparatus is provided. The transparent ultrasound probe module, which is provided inside the housing of the optical apparatus, according to one aspect of the present invention includes a lens provided on one side of an inside of the housing, formed of a transparent material, and disposed such that one surface thereof is exposed to the outside of the housing, a first matching layer provided on the other surface of the lens, and formed of a transparent material, a piezoelectric element layer provided on the first matching layer and including a plurality of transparent piezoelectric elements capable of interconverting an electrical signal and a mechanical signal, a first electrode layer and a second electrode layer formed of a transparent material, provided on one surface and the other surface of the piezoelectric element layer, respectively, and configured to transmit an electrical signal, circuit boards connected to each of the first electrode layer and the second electrode layer, formed of a bendable material, and configured to transmit and receive the electrical signal to and from the piezoelectric element, and a block layer provided on the second electrode layer, formed of a transparent material, and configured to remove ultrasound noise.

## Description

### CROSS-REFERENCE TO RELATED APPLICATION

This application claims priority to and the benefit of Korean Patent Application No. 10-2023-0103805 filed on August 8, 2023, the disclosure of which is incorporated herein by reference in its entirety.

### BACKGROUND

### 1. Field of the Invention

The present invention relates to a transparent ultrasound probe module and an optical apparatus having the same.

### 2. Discussion of Related Art

Ultrasound sensors (or ultrasound transducers) are sensors that enable the measurement of a physical distance to a target object and the acquisition of images of the object by utilizing the principle of converting electrical energy into acoustic energy through the properties of piezoelectric materials, transmitting the acoustic energy to the object, and converting the reflected acoustic energy back into an electrical signal.

Optics- and ultrasound-based imaging techniques have been steadily used in medical diagnostics due to their advantages over magnetic resonance imaging (MRI), X-ray, and computerized tomography (CT), including safety, accessibility, and real-time image monitoring.

Accordingly, research has been conducted on combining an existing ultrasound imaging system with an optical imaging system, combining the ultrasound imaging system with an optical coherence tomography imaging system, or combining the ultrasound imaging system with a fluorescence imaging system.

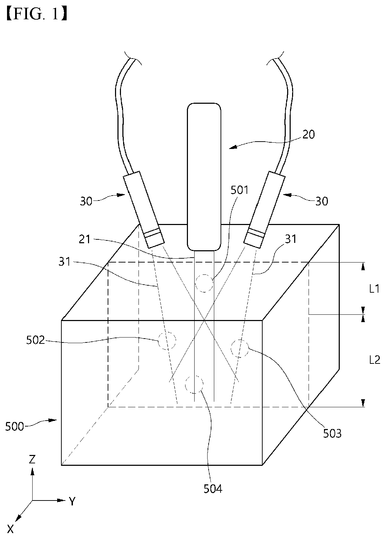

FIG. 1 is a view illustrating a state in which an object is scanned through a conventional opaque ultrasound sensor and an optical instrument. A conventional ultrasound sensor 20 is used in an opaque state, and thus, when the ultrasound sensor 20 is used in conjunction with an optical instrument 30, the optical instrument 30 cannot pass through the opaque ultrasound sensor 20 and is therefore disposed off an axis (un-axis insertion) of the ultrasound sensor as illustrated in FIG. 1.

Such un-axis insertion is disadvantageous in capturing images for various reasons. For example, there have been problems such as a misalignment of the system, an increase in complexity, an increase in system size, a decrease in signal-to-noise ratio (SNR), and a limited viewing angle.

Further, there have been difficulties in accurate diagnosis due to inconsistencies between optical scan data and ultrasound scan data.

For example, referring to FIG. 1, among a first material 501, a second material 502, a third material 503, and a fourth material 504 that are present in a specimen 500, the first material 501 and a portion of the fourth material 504 are detected by an ultrasound beam 21. However, there is a problem that the first material 501 located vertically below the ultrasound sensor 20 is not detected by an optical beam 31.

In the case of an optical apparatus that scans an object using the conventional opaque ultrasound sensor and the optical instrument, a light source cannot pass through the ultrasound sensor. Thus, in order to separate an optical path and an ultrasound signal path, ancillary elements, such as a prism and silicon oil, are inevitably disposed. This results in a problem that the size of a system significantly increases.

Meanwhile, in order to solve the problem of the opaque ultrasound sensor, in US Patent No. 8,784,321, a portion of a cross section of the opaque ultrasound sensor is perforated to form an optical path, so that the optical path and an ultrasound path are located on the same axis. However, even in this case, since light can pass through only the portion of the cross section of the ultrasound sensor, the problem due to light non-transmittance of the ultrasound sensor cannot be sufficiently solved.

Meanwhile, in order to solve the problem of image capturing due to the opaque ultrasound sensor, a transparent ultrasound sensor has been developed in recent years. However, some transparent ultrasound sensors still have limitations in obtaining a high SNR and high-resolution photoacoustic images due to low transparency, low sensitivity, or wide focusing.

The inventors of the present invention have proposed a lithium niobate (LNO)-based single crystal transparent ultrasound sensor structure and a method for manufacturing the same in Korean Patent Publication No. 10-2021-0034466 ("TRANSPARENT ULTRASONIC SENSOR AND MANUFACTURING METHOD THEREOF"), and based on this, the inventors of the present invention have proposed an optical multi imaging system for a single element type transparent ultrasound sensor in Korean Patent Publication No. 10-2022-0029003 ("ULTRASONIC-OPTICAL MULTI IMAGING SYSTEM BASED ON TRANSPARENT ULTRASONIC SENSOR").

However, since the above ultrasound sensor structure is composed of a single element, the amount of data that can be processed per hour with a single element is limited. Accordingly, there is a growing need for an optical apparatus that can improve image quality and provide a wider viewing angle by increasing the amount of data while maintaining the advantages of a transparent ultrasound sensor that can process data in real time by adopting a transparent ultrasound sensor with a multi-element structure.

### [Prior Art Document]

### [Patent Documents]

(Patent Document 0001) Korean Patent Publication No. 10-2021-0034466
(Patent Document 0002) Korean Patent Publication No. 10-2022-0029003

### SUMMARY OF THE INVENTION

The present invention is directed to providing an optical apparatus capable of improving a signal-to-noise ratio (SNR) and reducing a size of a device by using a transparent ultrasound sensor enabling coaxialization of an ultrasound path and an optical path.

The present invention is also directed to providing an optical apparatus including an ultrasound sensor with high transparency and sensitivity.

The present invention is also directed to providing an optical apparatus capable of improving image quality and providing a wide viewing angle by employing multiple piezoelectric elements.

It should be noted that objects of the present invention are not limited to the above-described objects, and other objects of the present invention will be apparent to those skilled in the art from the following descriptions.

A transparent ultrasound probe module, which is provided inside a housing of an optical apparatus, according to one aspect of the present invention includes a lens provided on one side of an inside of the housing, formed of a transparent material, and disposed such that one surface thereof is exposed to the outside of the housing, a first matching layer provided on the other surface of the lens, and formed of a transparent material, a piezoelectric element layer provided on the first matching layer and including a plurality of transparent piezoelectric elements capable of interconverting an electrical signal and a mechanical signal, a first electrode layer and a second electrode layer formed of a transparent material, provided on one surface and the other surface of the piezoelectric element layer, respectively, and configured to transmit an electrical signal, circuit boards connected to each of the first electrode layer and the second electrode layer, formed of a bendable material, and configured to transmit and receive the electrical signal to and from the piezoelectric element, and a block layer provided on the second electrode layer, formed of a transparent material, and configured to remove ultrasound noise.

The transparent ultrasound probe module may further include a second matching layer formed of a transparent material, between the first matching layer and the lens.

The circuit boards may be formed of a transparent material.

The circuit boards may include a ground board (ground fpcb) and a signal board (signal fpcb).

The ground fpcb may be connected to the first electrode layer, between the first electrode layer and the first matching layer, and the signal fpcb may be connected to the second electrode layer, between the second electrode layer and the block layer.

The circuit boards may include a gold foil performing a grounding function and a signal fpcb.

The gold foil plate may be connected to the first electrode layer, between the first electrode layer and the first matching layer, and the signal fpcb may be connected to the second electrode layer, between the second electrode layer and the block layer.

The block layer may be formed to have a wider surface than each of the piezoelectric element layer and the second electrode layer, and the signal fpcb may be disposed on the piezoelectric element layer, which is located on an upper surface of the block layer, and on a portion of the upper surface of the block layer protruding from the second electrode layer, and connected to the second electrode layer through a conductive material.

The plurality of piezoelectric elements constituting the piezoelectric element layer may be disposed in a row.

The plurality of piezoelectric elements may be disposed in a row, and arranged in the shape of an arch protruding convexly upward.

The plurality of piezoelectric elements may be disposed in a row, and are arranged in a U-shape that is concave upward.

The plurality of piezoelectric elements constituting the piezoelectric element layer may be disposed in a grid shape horizontally and vertically.

The plurality of piezoelectric elements constituting the piezoelectric element layer may be disposed in a circular shape.

The plurality of piezoelectric elements may be formed in the form of rings with different diameters and disposed in an annular shape.

The plurality of piezoelectric elements may be disposed in a hemispherical shape having a constant curvature.

The matching layer and the block layer may be formed of one or more materials from among plastics, silicone, glass, and epoxy, or formed of a composite in which a powder having a diameter of several nanometers to hundreds of micrometers is mixed with one of the materials.

The lens may have the shape of one of a concave lens, a convex lens, and a flat lens.

According to another aspect of the present invention, there is provided an optical/ultrasound optical apparatus including a housing, the above-described transparent ultrasound probe module provided on one side of an inside of the housing, and an optical instrument provided on the other side of the inside of the housing and disposed coaxially with the transparent ultrasound probe module in a longitudinal direction of the housing.

Here, the optical/ultrasound optical apparatus may further include an optical lens provided inside the housing, and disposed between the optical instrument and the transparent ultrasound probe module.

The optical instrument may include a camera.

The optical instrument may include a light source.

### BRIEF DESCRIPTION OF THE DRAWINGS

The above and other objects, features, and advantages of the present invention will become more apparent to those of ordinary skill in the art by describing exemplary embodiments thereof in detail with reference to the accompanying drawings, in which:
FIG. 1 is a view illustrating a state in which a specimen is scanned using a conventional opaque ultrasound probe and an optical instrument;
FIG. 2 is a set of views illustrating photoacoustic tomographic images and ultrasound tomographic images that can be obtained when the ultrasound probe and the optical instrument are disposed coaxially and non-coaxially;
FIG. 3 is a perspective view illustrating an optical apparatus according to one embodiment of the present invention;
FIG. 4 is a cross-sectional view illustrating the optical apparatus according to one embodiment of the present invention;
FIG. 5 is a view illustrating a state in which the optical apparatus according to one embodiment of the present invention scans an object;
FIG. 6 is a view illustrating an ultrasound probe module provided in an optical apparatus according to a first embodiment of the present invention;
FIG. 7 a view illustrating a cross section of the ultrasound probe module along line I-I' in FIG. 6, along with an enlarged view of a portion of the corresponding cross section;
FIG. 8 a view illustrating a cross section of the ultrasound probe module along line II-II' in FIG. 6, along with an enlarged view of a portion of the corresponding cross section;
FIG. 9 is a view illustrating an ultrasound probe module of an optical apparatus according to a second embodiment of the present invention;
FIG. 10 is a view illustrating a cross section of the ultrasound probe module according to the second embodiment along line III-III' in FIG. 9, along with an enlarged view of a portion of the corresponding cross section;
FIG. 11 is a view illustrating a cross section of the ultrasound probe module according to the second embodiment along line IV-IV' in FIG. 9.
FIG. 12 is a view illustrating a cross section of an ultrasound probe module of an optical apparatus according to a third embodiment of the present invention viewed from the same direction as in FIG. 10, along with an enlarged view of a portion of the corresponding cross section;
FIG. 13 is a view illustrating an ultrasound probe module of an optical apparatus according to a first modified example of arrangement of piezoelectric elements of the optical apparatus according to the present invention;
FIG. 14 is a view illustrating an ultrasound probe module of an optical apparatus according to a second modified example of arrangement of piezoelectric elements of the optical apparatus according to the present invention;
FIG. 15 is a view illustrating an ultrasound probe module of an optical apparatus according to a third modified example of arrangement of piezoelectric elements of the optical apparatus according to the present invention;
FIG. 16 is a view illustrating an ultrasound probe module of an optical apparatus according to a fourth modified example of arrangement of piezoelectric elements of the optical apparatus according to the present invention; and
FIG. 17 is a view illustrating an ultrasound probe module of an optical apparatus according to a fifth modified example of arrangement of piezoelectric elements of the optical apparatus according to the present invention.

### DETAILED DESCRIPTION OF EXEMPLARY EMBODIMENTS

Hereinafter, with reference to the accompanying drawings, the embodiments of the present invention will be described in detail so that those of ordinary skill in the art to which the present invention pertains can easily implement the present invention. The present invention may be implemented in many different forms and is not limited to the embodiments described herein. In order to clearly describe the present invention, parts irrelevant to the description are omitted from the drawings, and the same reference numerals are assigned to the same or similar components throughout the specification.

The words and terms used in the present specification and claims are not limited and interpreted to the conventional or dictionary meanings, and in accordance with the principle that the inventors may define terms and concepts in order to describe their invention in the best way, should be interpreted with meanings and concepts consistent with the technical spirit of the present invention.

In the present specification, terms such as "include" or "have" are intended to describe the presence of features, numbers, steps, operations, components, parts, or a combination thereof described in the specification, but it is to be understood that they do not preclude the possibility of the presence or addition of one or more other features, numbers, steps, operations, components, parts, or a combination thereof.

A case in which a component is at the "front," "rear," "top," or "bottom" of another component includes, unless otherwise specified, not only a case in which the component is in direct contact with the other component and is disposed at the "front," "rear," "top," or "bottom," but also cases in which other components are disposed therebetween. In addition, a case in which a component is "connected" to another component includes, unless otherwise specified, not only cases in which the components are directly connected to each other, but also cases in which the components are indirectly connected to each other.

An optical apparatus according to one embodiment of the present invention is an optical apparatus for scanning an object to be diagnosed in a state in which an ultrasound probe module formed to be transparent and an optical instrument are arranged coaxially.

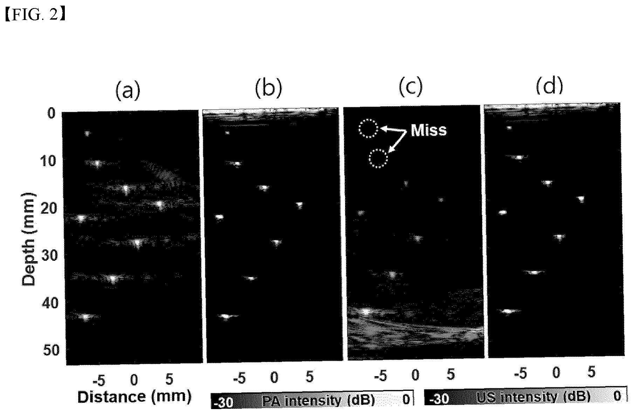

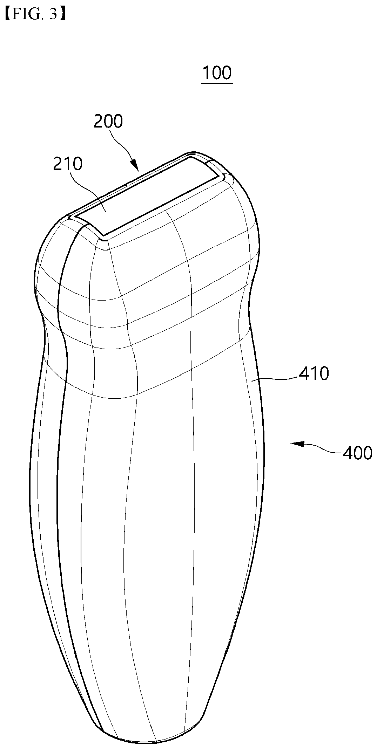

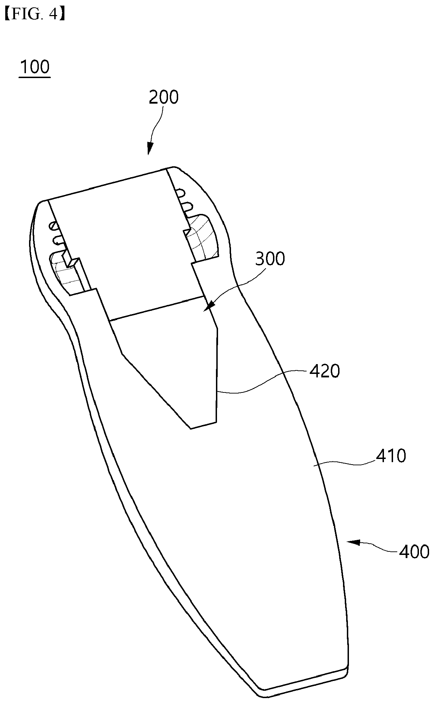

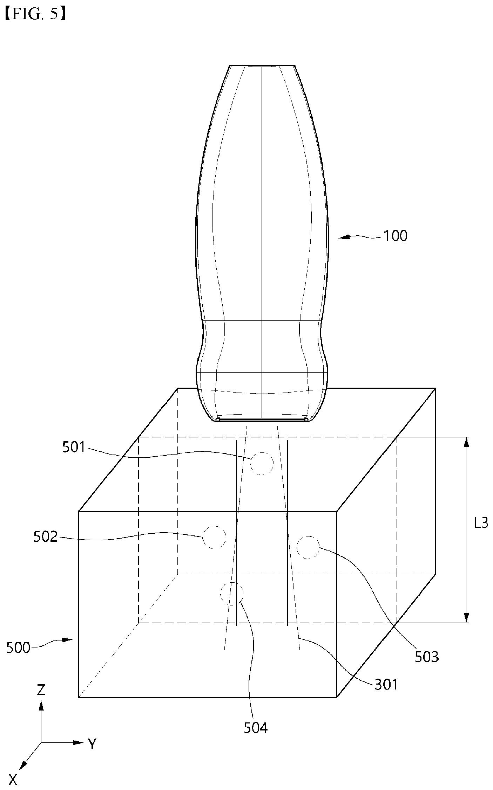

FIG. 1 is a view illustrating a state in which a specimen is scanned using a conventional opaque ultrasound probe and an optical instrument. FIG. 2 is a set of views illustrating photoacoustic scan data and ultrasound scan data that can be obtained when the ultrasound probe and the optical instrument are disposed coaxially and non-coaxially. FIG. 3 is a perspective view illustrating an optical apparatus according to one embodiment of the present invention. FIG. 4 is a cross-sectional view illustrating the optical apparatus according to one embodiment of the present invention. FIG. 5 is a view illustrating a state in which the optical apparatus according to one embodiment of the present invention scans a specimen. As used herein, a specimen 500 may be any object that can be inspected by an optical/ultrasound probe. For example, the specimen 500 may be a human body tissue or may be a biological tissue of another living organism.

Hereinafter, an optical apparatus 100 according to one embodiment of the present invention will first be described, and then structures of an ultrasound probe module 200 and an optical instrument 300 constituting the optical apparatus 100 will be described.

In FIG. 5, the transparent ultrasound probe module, the optical instrument, and the specimen 500 according to one embodiment of the present invention are illustrated. The specimen 500 illustrated in FIG. 5 is illustrated such that its core is oriented in a negative direction of a z-axis, and its outer surface is oriented in a positive direction of the z-axis, when viewed in FIG. 5.

Referring to FIGS. 3 to 5, in the optical apparatus 100 according to one embodiment of the present invention, the ultrasound probe module 200 and the optical instrument 300 may be coaxially coupled to each other inside a housing 400.

A side surface of the housing 400 may be formed in the shape of a curved surface that is curved in a height direction to facilitate gripping during use by a user.

In addition, an opening may be formed in one side of the housing 400 to allow beams emitted from the ultrasound probe module 200 and the optical instrument 300 disposed inside the housing 400 to escape to the outside.

The optical apparatus 100 according to one embodiment of the present invention may be provided as a wireless apparatus as illustrated in FIG. 3, but is not limited thereto, and may be provided as a wired apparatus.

When the optical apparatus 100 is provided as a wireless apparatus, the other side of the housing 400 may be formed to be sealed, whereas when the optical apparatus 100 is provided as a wired apparatus, an opening for a cable to be connected and a separate support for stably supporting the cable may be formed on the other side of the housing 400.

Further, referring to FIG. 4, a coupling holder 420 that allows the optical instrument 300 to be stably fixed inside the housing while coupled to the ultrasound probe module 200 may be provided inside the housing 400.

The coupling holder 420 may be configured as a part of a housing body 410 and formed to surround the optical instrument 300, and may protrude from the optical instrument 300 at one side thereof and support the ultrasound probe module 200 at the other side thereof.

The ultrasound probe module 200 is located on one side of the inside of the housing 400, and one side of the ultrasound probe module 200 may be exposed to the outside through the opening provided at one side of the housing 400.

A lens 210 may be located on one side portion of the ultrasound probe module 200 which may be exposed to the outside.

As illustrated in FIG. 3, one surface of the lens 210 of the ultrasound probe module 200 may be exposed to the outside through the opening of the housing 400, and the remaining portion of the lens 210 and the ultrasound probe module 200 may be located inside the housing 400.

Referring to FIG. 4, the optical instrument 300 may be disposed on the other side of the ultrasound probe module 200. In this case, in order for the ultrasound probe module 200 to be coaxially coupled with the optical instrument 300, the ultrasound probe module 200 may be formed of a transparent material that does not obstruct an optical path.

At this time, the optical instrument 300 may be provided as a camera for photographing light entering the inside of the housing 400 from the outside, or may be provided as a light source for emitting light to the outside from the inside of the housing 400.

However, the optical instrument 300 is not limited thereto, and may be provided as an instrument that has both camera and light source functions, or may be formed in a form in which a plurality of instruments are coupled.

In addition, although not illustrated in the drawing, an optical system for setting an optical path to be suitable for a purpose may be disposed between the ultrasound probe module 200 and the optical instrument 300.

In this case, the optical system may include convex and concave lenses that can change the path of light, as well as cylindrical lenses, ball lenses, beam shapers, beam diffusers, and the like.

According to one embodiment of the present invention according to the above configuration, since the ultrasound probe module 200 and the optical instrument 300 are coaxially disposed in the housing 400, an ultrasound beam 201 emitted from the ultrasound probe module 200 to the specimen 500 for scanning and an optical beam 301 emitted from the optical instrument 300 to the specimen 500 for scanning propagate straight in parallel directions, unlike in FIG. 1.

Accordingly, unlike in FIG. 1 in which images formed by the ultrasound beam 21 and the optical beam 31 respectively emitted from the conventional ultrasound sensor 20 and the optical instrument 30 reach different positions on the outer surface of the specimen 500, when using the optical apparatus 100 according to one embodiment of the present invention, the image formed by the ultrasound beam 201 and the image formed by the optical beam 301 can reach the same position on the outer surface of the specimen 500.

Accordingly, unlike with the conventional optical apparatus 100, in which the optical beam 31 cannot reach a region reached by the ultrasound beam 21 within a certain depth L1 from the outer surface of the specimen 500, with the optical apparatus according to one embodiment of the present invention, as illustrated in FIG. 5, both ultrasound image information and optical image information can be obtained throughout an entire area L3 in a depth direction of the specimen 500.

Further, it is also possible to solve the problem of the low signal-to-noise ratio that occurred in the conventional device as the ultrasound beam 21 and the optical beam 31 propagate along different paths.

This effect can also be confirmed in photoacoustic scan data (in FIG. 2A) and ultrasound scan data (in FIG. 2B) obtained when the ultrasound probe and the optical instrument are coaxially disposed, as in the optical apparatus according to one embodiment of the present invention.

FIG. 2A is photoacoustic scan data obtained in a state in which the ultrasound probe and the optical instrument are coaxially disposed, and FIG. 2B is ultrasound scan data obtained in a state in which the ultrasound probe and the optical instrument are coaxially disposed. FIG. 2C is photoacoustic scan data obtained in a state in which the ultrasound probe and the optical instrument are non-coaxially disposed, and FIG. 2D is ultrasound scan data obtained in a state in which the ultrasound probe and the optical instrument are non-coaxially disposed.

Comparing FIGS. 2A and 2C, it can be confirmed that in FIG. 2C, which is the scan data obtained when the ultrasound probe and the optical instrument are non-coaxially disposed, data from a shallow depth portion is missing.

In addition, in the scan data illustrated in FIG. 2C, it can be confirmed that much noise occurs in a deeper portion.

As described above, when the ultrasound probe and the optical instrument are coaxially coupled, the photoacoustic scan data (in FIG. 2A) matches the ultrasound scan data (FIG. 2B), whereas, when the ultrasound probe and the optical instrument are non-coaxially coupled, the photoacoustic scan data (FIG. 2C) and the ultrasound scan data (FIG. 2D) do not match.

Hereinafter, the structure of the ultrasound probe module 200 provided in the optical apparatus 100 according to the present invention will be described with reference to different drawings.

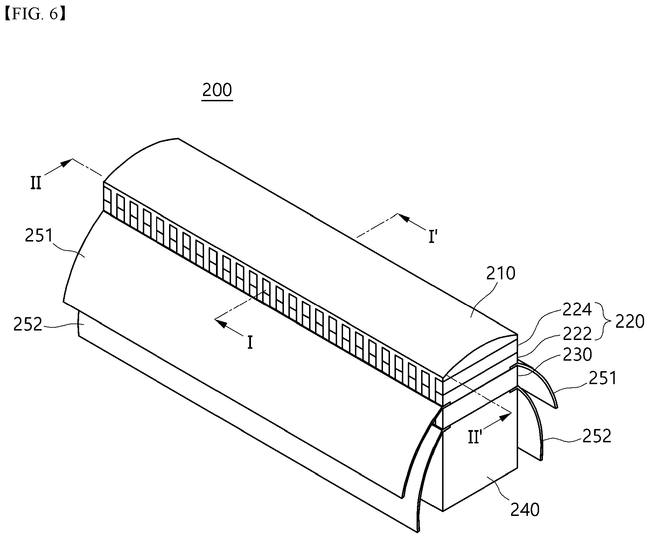

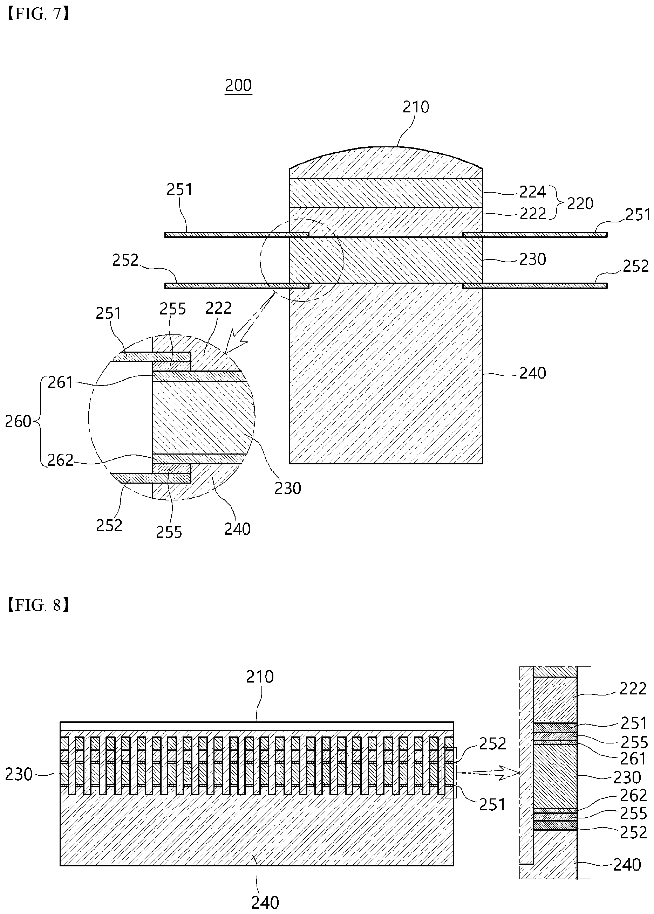

FIG. 6 is a view illustrating an ultrasound probe module provided in an optical apparatus according to a first embodiment of the present invention. FIG. 7 is a view illustrating a cross section of the ultrasound probe module along line I-I' in FIG. 6, along with an enlarged view of a portion of the corresponding cross section. FIG. 8 is a view illustrating a cross section of the ultrasound probe module along line II-II' in FIG. 6, along with an enlarged view of a portion of the corresponding cross section.

Referring to FIGS. 3 and 6, an ultrasound probe module 200, which may be installed in the optical apparatus according to one embodiment of the present invention, is located at one side portion the housing 400, in which an opening is formed, and the lens 210 is provided on one side of the ultrasound probe module 200 such that one surface of the lens 210 is exposed to the outside through the opening of the housing 400.

Here, the lens 210 may be formed to have any one shape of a concave lens, a convex lens, and a flat lens according to the purpose.

The lens 210 of the ultrasound probe module 200 according to one embodiment of the present invention may be formed of a silicon material, and may also be formed of a water-based gel, plastic, or epoxy resin.

A matching layer 220 may be disposed on the other surface of the lens of the ultrasound probe module 200 according to one embodiment of the present invention, and a piezoelectric element layer 230 formed of one or more piezoelectric elements may be disposed on the other surface of the matching layer 220.

The matching layer 220 is for reducing a difference in acoustic impedance with a medium onto which an ultrasound signal generated by the piezoelectric element constituting the piezoelectric element layer 230 is emitted, that is, the specimen 500.

That is, when an ultrasound signal is generated by the operation of the piezoelectric element, in order for the ultrasound signal to be transmitted efficiently in water, biological tissue, or other media other than air, an acoustic impedance of the medium must be adjusted as much as possible to minimize the loss of ultrasound energy.

The matching layer 220 of the present example may be a focused type, equipped with an acoustic lens that enables adjustment of the focusing of both light and the ultrasound signal.

In this case, due to the focusing function of the matching layer 220, the ultrasound signal, which is reflected by the specimen 500 and incident on the transparent ultrasound probe module 200, may be accurately focused at a desired position of the piezoelectric element.

Thus, by the focusing function of the matching layer 220, an ultrasound image obtained by the ultrasound signal output from the piezoelectric element layer 230 can be focused so that a clear ultrasound image can be obtained.

Accordingly, the clarity of an image obtained by the operation of the transparent ultrasound probe module 200 can be improved, and thus a clear image of a desired portion of the specimen 500 onto which the ultrasound signal is emitted can be obtained.

In addition, when the matching layer 220 uses an acoustic lens, a surface thereof may have a constant curve and increased transparency so that the amount of loss of an ultrasound signal, which is emitted onto or reflected from the specimen 500 when transmitting and receiving the ultrasound signal can be reduced.

Further, an additional transmission film or a blocking film may be formed in the matching layer 220 as desired to allow only a signal of a desired wavelength to be transmitted or blocked.

In the case of the matching layer 220 according to one embodiment of the present invention, two layers, a first matching layer 222 and a second matching layer 224, may be formed to achieve the above purpose.

The acoustic lens provided in the matching layer 220 may be formed of a material that takes into account an acoustic impedance, so that ultrasound waves are transmitted with high efficiency, that is, the reflection does not occur between layers.

For example, the matching layer 220 may be formed of a transparent plastic, silicone, glass, or an epoxy resin, and may also be formed of a composite in which a powder having a diameter of several nanometers to hundreds of micrometers is mixed with one of the above materials.

The piezoelectric element layer 230 provided on the other side of the matching layer 220 may be formed of one or more piezoelectric elements.

The piezoelectric element may produce a piezoelectric effect, which causes polarization when a mechanical force is applied to the element to generate electricity, and a converse piezoelectric effect, which causes mechanical motion of contraction and relaxation when a voltage is applied to the element.

The ultrasound probe module 200 according to one embodiment of the present invention may utilize the piezoelectric effect and the converse piezoelectric effect to transmit ultrasound waves toward the specimen 500, and receive ultrasound waves reflected from the specimen 500 to generate corresponding electrical signals.

The number of piezoelectric elements constituting the piezoelectric element layer 230 may vary according to the size of the ultrasound probe module 200. For example, the piezoelectric element layer 230 may include 64 piezoelectric elements or 1024 piezoelectric elements.

Here, a plurality of piezoelectric elements may be manufactured through a process of mechanically cutting a relatively large-sized piezoelectric element, such as using a dicing saw or laser cutting.

At this time, the piezoelectric element may be formed of lithium niobate (LNO), and may include LNO as well as transparent piezoelectric materials exhibiting piezoelectric properties, such as lead magnesium niobate titanium (PMN-PT), polyvinylidene fluoride (PVDF), and polyvinylidene fluoride-trifluoroethene (PVDF-TrFE).

Referring to FIGS. 7 and 8, electrode layers 260 for transmitting an electrical signal may be formed on one surface and the other surface of the piezoelectric element layer 230.

The electrode layers 260 may include a first electrode layer 261 disposed between one surface of the piezoelectric element layer 230 and the other surface of the first matching layer 222, and a second electrode layer 262 disposed between the other surface of the piezoelectric element layer 230 and a block layer 240.

The first electrode layer 261 and the second electrode layer 262 may be formed by being respectively deposited on upper and lower surfaces of each piezoelectric element constituting the piezoelectric element layer 230. In addition, the first electrode layer 261 and the second electrode layer 262 may be insulated from each other to prevent an electrical short circuit therebetween.

The first electrode layer 261 and the second electrode layer 262 may be formed of a transparent material as in other components of the ultrasound probe module 200 according to one embodiment of the present invention.

More specifically, the first electrode layer 261 and the second electrode layer 262 may each be formed of a transparent and conductive material, such as silver nanowire (AgNW), copper nanowire (CuNW), gold nanowire (AuNW), indium-tin-oxide (ITO), carbon nanotubes, and graphene.

In addition, the first electrode layer 261 and the second electrode layer 262 may each be formed to a thickness of, for example, less than 100 nm.

Meanwhile, referring to FIGS. 6 to 8, the block layer 240 may be disposed on the other surface of the second electrode layer 262 of the ultrasound probe module 200 according to one embodiment of the present invention.

The block layer 240 may perform a damping role for removing ultrasound noise generated by the ultrasound probe module 200 according to one embodiment of the present invention.

The ultrasound beam 201 generated by the piezoelectric element layer 230 propagates forward from the optical apparatus 100 in which the specimen 500 is located, but also propagates backward opposite thereto.

In this case, unlike the forward propagating ultrasound beam 201, the backward propagating ultrasound beam may act as noise during the scanning of the specimen 500 by the ultrasound probe module 200.

In addition, when the ultrasound beam 201 propagating forward from the optical apparatus 100 is reflected by the specimen 500 and returned back to the ultrasound probe module 200, the ultrasound beam 201 may be reflected again by passing through the matching layer 220 and the piezoelectric element layer 230. Accordingly, unnecessary signal interference may occur.

The block layer 240 may serve to prevent such unnecessary signal interference and attenuate the ultrasound signal directed to the other side of the piezoelectric element layer 230, i.e., backward from the optical apparatus 100.

Here, since the optical beam should be able to pass through the other side of the block layer 240, the block layer 240 may also be formed of a transparent material.

More specifically, the block layer 240 may be formed of a plastic, silicone, glass, or an epoxy resin, and may also be formed of a composite in which a powder having a diameter of several nanometers to hundreds of micrometers is mixed with one of the above materials in order to increase attenuation performance.

Referring to FIGS. 6 to 8, circuit boards 251 and 252 may be connected to the first electrode layer 261 and the second electrode layer 262 of the ultrasound probe module 200 according to one embodiment of the present invention, respectively.

The circuit boards may be formed of flexible printed circuit boards, which need to fit into a tight space within the housing 400 of the optical apparatus 100 and thus are typically thin and flexible.

In the case of the ultrasound probe module 200 according to one embodiment of the present invention, the circuit boards may include a ground board (ground fpcb) 251 responsible for grounding and a signal board (signal fpcb) 252 responsible for signal transmission.

The signal fpcb 252 may have lines and pads corresponding to respective piezoelectric elements for signal exchange therewith, and each line and pad may be configured to correspond one-to-one with each piezoelectric element.

Here, the ground fpcb 251 may be connected to one side of the first electrode layer 261 and located between the first electrode layer 261 and the first matching layer 222, and the signal fpcb 252 may be connected to the other side of the second electrode layer 262 and located between the second electrode layer 262 and the block layer 240.

The ground fpcb 251 and the signal fpcb 252 may also be formed of an opaque material.

When the ground fpcb 251 and the signal fpcb 252 are made of an opaque material, a portion corresponding to a center portion of the piezoelectric element layer 230 through which the ultrasound beam 201 or the optical beam 301 passes may be formed by opening the ground fpcb 251 and the signal fpcb 252, and the ground fpcb 251 and the signal fpcb 252 may be attached to edges of the piezoelectric element layer 230.

When the ground fpcb 251 and the signal fpcb 252 are formed of a transparent material, the portion corresponding to the center portion of the piezoelectric element layer 230 may be formed without opening the ground fpcb 251 and the signal fpcb 252.

In this case, referring to FIGS. 7 and 8, gold foils 255 of the circuit boards may be formed to expose a portion in which the ground fpcb 251 and the first electrode layer 261 are connected and a portion in which the signal fpcb 252 and the second electrode layer 262 are connected.

Except for the portion in which the ground fpcb 251 and the first electrode layer 261 are connected, the portion in which the signal fpcb 252 and the second electrode layer 262 are connected, and connection portions of the ground fpcb 251 and the signal fpcb 252, which are connected to a connector of the optical apparatus 100 connected to each circuit board, the remaining portions of the ground fpcb 251 and the signal fpcb 252 may be formed to be insulated.

In this case, the ground fpcb 251 and the signal fpcb 252 may each be formed to a thickness of less than 50 µm, and the gold foil 255 may be formed to a thickness of less than 100 nm.

The above stacked structure may be applied to all the piezoelectric elements constituting each piezoelectric element layer 230, and referring to FIGS. 6 and 8, when the plurality of piezoelectric elements are disposed in a row, the stacked structure and each of the piezoelectric elements may be arranged at a predetermined interval.

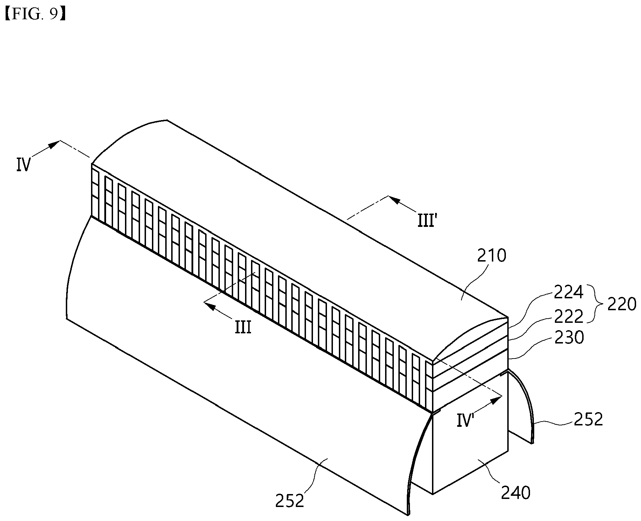

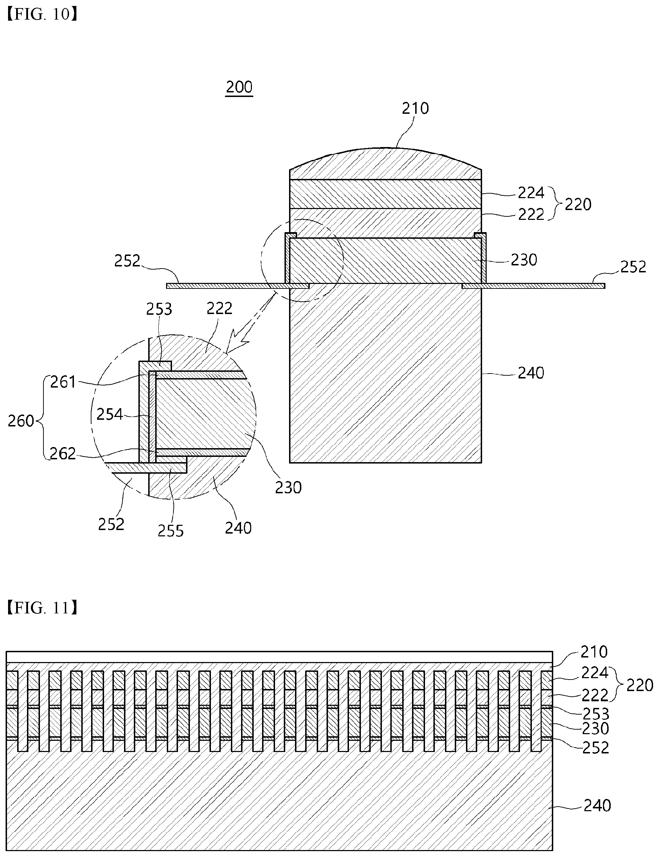

FIG. 9 is a view illustrating an ultrasound probe module provided in an optical apparatus according to a second embodiment of the present invention. FIG. 10 is a view illustrating a cross section of the ultrasound probe module according to the second embodiment along line III-III' in FIG. 9, along with an enlarged view of a portion of the corresponding cross section. FIG. 11 is a view illustrating a cross section of the ultrasound probe module according to the second embodiment along line IV-IV' in FIG. 9, along with an enlarged view of a portion of the corresponding cross section. Hereinafter, in describing another embodiment of the present invention, detailed description of components identical to those of the above-described embodiment will be omitted, and various embodiments of the present invention will be described focusing on components that are different from the above-described embodiment.

Referring to an ultrasound probe module 200 according to the second embodiment of the present invention illustrated in FIGS. 9 to 11, the matching layer 220, the piezoelectric element layer 230, and the block layer 240 are sequentially disposed from the lens 210 to the other side of the ultrasound probe module 200, which is the same as the ultrasound probe module 200 according to the first embodiment illustrated in FIGS. 6 to 8.

However, unlike the ultrasound probe module 200 illustrated in FIGS. 6 to 8, in the case of a modified example of the ultrasound probe module 200 illustrated in FIGS. 9 to 11, the circuit board may be configured with only the signal fpcb 252 without the ground fpcb.

Here, referring to FIG. 10, in the modified example of the ultrasound probe module 200 according to one embodiment of the present invention, a ground metal foil 253 replacing the ground fpcb with gold foil may be provided.

One end portion of the ground metal foil 253 may be connected to the first electrode layer 261, and the other end portion thereof may be connected to the signal fpcb 252.

Here, referring to FIG. 10, an insulating member 254 may be provided in a space between the ground metal foil 253 and the piezoelectric element layer 230, the second electrode layer 262, and the gold foil 255 of the signal fpcb 252 to prevent a short circuit between the ground metal foil 253 and other components.

In the ultrasound probe module 200 according to one embodiment of the present invention, an adhesive member, such as epoxy, may be applied to the circuit board or the gold foil and then pressure may be applied to adhere the circuit board or the gold foil to the piezoelectric element layer 230.

Here, an adhesive layer formed by the adhesive member used for adhesion is formed to a thickness equal to that of the circuit board or gold foil being adhered.

Here, since the adhesive member may affect ultrasound scan data detected by the ultrasound probe module 200, the thinner the adhesive layer is formed, the more noise- or distortion-free ultrasound scan data can be obtained.

The circuit board may be formed to a thickness of 50 µm, but the gold foil may be formed to a thickness of less than 100 nm, so that when adopting a modified configuration such as the ultrasound probe module 200 illustrated in FIGS. 9 to 11, the adhesive layer can be formed to be thinner than that of the ultrasound probe module 200 illustrated in FIGS. 6 to 8.

Accordingly, it is possible to obtain more accurate ultrasound scan data when adopting the structure according to the corresponding modified example.

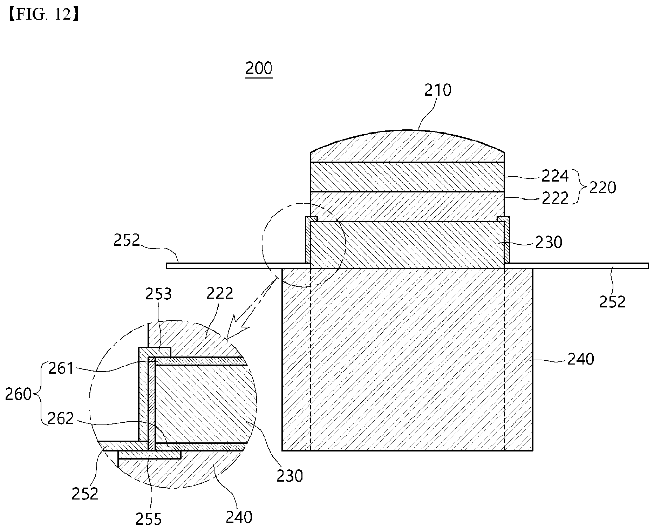

FIG. 12 is a view illustrating a cross section of an ultrasound probe module of an optical apparatus according to a third embodiment of the present invention viewed from the same direction as in FIG. 10, along with an enlarged view of a portion of the corresponding cross section.

Referring to FIG. 12, in the case of the third embodiment, the block layer 240 disposed on the other side of the piezoelectric element layer 230 may be formed to have a larger width than each of the piezoelectric element layer 230, the matching layer 220, and the lens 210.

Accordingly, unlike the ultrasound probe module 200 illustrated in FIGS. 6 to 11, the signal fpcb 252 does not interfere between the piezoelectric element layer 230 and the block layer 240, and may be connected to the piezoelectric element layer 230 at a side surface of the piezoelectric element layer 230.

In this case, for electrical connection between the piezoelectric element layer 230 and the signal fpcb 252, the gold foil 255 protruding from the signal fpcb 252 may be disposed between the piezoelectric element layer 230 and the block layer 240.

In this case, the gold foil may be formed to a thickness of less than 100 nm, whereas the signal fpcb may be formed to a thickness of 50 µm, so that, in the corresponding modified example, since the signal fpcb 252 interferes between the piezoelectric element layer 230 and the block layer 240, the effect from the transmission and reception of the ultrasound wave can be minimized.

Further, since the corresponding modified example employs a configuration that replaces the ground fpcb with the ground metal foil 253 as in the case of the ultrasound probe module 200 illustrated in FIGS. 9 to 11, the adhesive layer can be formed to be thinner than in the configuration of the ultrasound probe module 200 illustrated in FIGS. 6 to 8.

The above stacked structure of the ultrasound probe module 200 may be equally applied to additional modified examples in which the arrangement of the piezoelectric elements constituting the piezoelectric element layer 230 is changed.

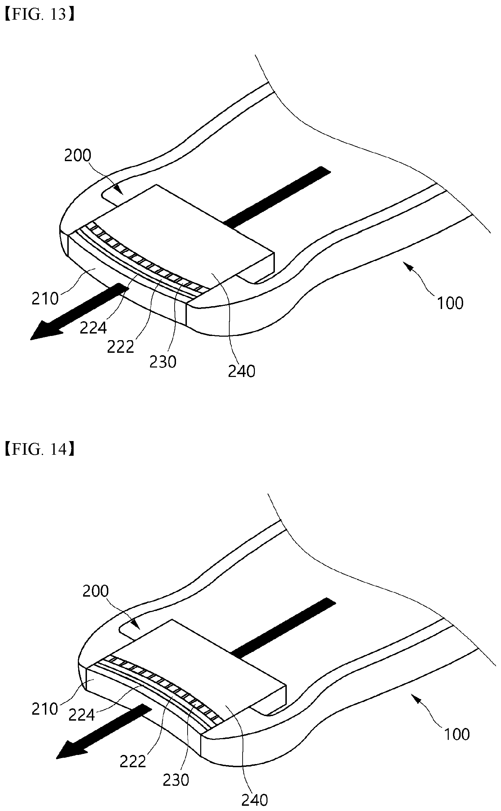

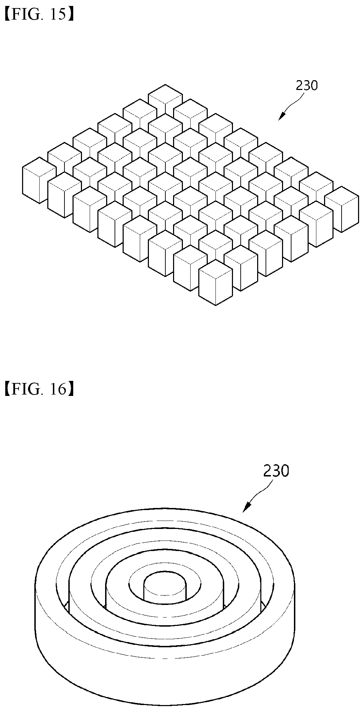

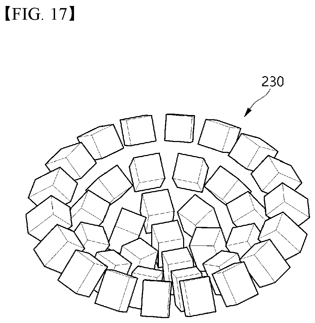

FIG. 13 is a view illustrating an ultrasound probe module of an optical apparatus according to a first modified example of arrangement of piezoelectric elements of the optical apparatus according to the present invention. FIG. 14 is a view illustrating an ultrasound probe module of an optical apparatus according to a second modified example of arrangement of piezoelectric elements of the optical apparatus according to the present invention. FIG. 15 is a view illustrating an ultrasound probe module of an optical apparatus according to a third modified example of arrangement of piezoelectric elements of the optical apparatus according to the present invention. FIG. 16 is a view illustrating an ultrasound probe module of an optical apparatus according to a fourth modified example of arrangement of piezoelectric elements of the optical apparatus according to the present invention. FIG. 17 is a view illustrating an ultrasound probe module of an optical apparatus according to a fifth modified example of arrangement of piezoelectric elements of the optical apparatus according to the present invention.

Referring to FIG. 13, the piezoelectric elements constituting the piezoelectric element layer 230 may be arranged in a convex shape.

When the piezoelectric element layer 230 is formed in a convex shape, the lens 210, the matching layer 220, and the block layer 240 may also be formed in a convex shape accordingly.

This case is advantageous because the ultrasound probe module 200 can emit a signal over a larger region.

Even when the piezoelectric element layer 230, the lens 210, the matching layer 220, and the block layer 240 of the ultrasound probe module 200 are formed in a convex shape as described above, the stacking order and transparency in the ultrasound probe module 200 and the positional relationship with the optical instrument 300 in the housing 400 may be the same as in the case in which the arrangement of the piezoelectric element layer 230 is not deformed, which has been described above.

Accordingly, as illustrated in FIG. 13, even in the case of the first modified example of the piezoelectric element layer 230 of the ultrasound probe module 200 according to one embodiment of the present invention, light can pass through the ultrasound probe module 200 from the optical instrument 300 in the housing and propagate to the outside of the housing.

Referring to FIG. 14, the piezoelectric elements constituting the piezoelectric element layer 230 may be disposed in a concave shape.

When the piezoelectric element layer 230 is formed in a concave shape, the lens 210, the matching layer 220, and the block layer 240 may also be formed in a concave shape accordingly.

This case is advantageous because the ultrasound probe module 200 can emit a signal by focusing the signal on a relatively narrow region.

Even when the piezoelectric element layer 230, the lens 210, the matching layer 220, and the block layer 240 of the ultrasound probe module 200 are formed in a convex shape as described above, as in FIG. 13, the stacking order and transparency in the ultrasound probe module 200 and the positional relationship with the optical instrument 300 in the housing 400 may be formed to be the same as in the case in which the arrangement of the piezoelectric element layer 230 is not deformed, which has been described above.

Accordingly, as illustrated in FIG. 14, even in the case of the second modified example of the piezoelectric element layer 230 of the ultrasound probe module 200 according to one embodiment of the present invention, light can pass through the ultrasound probe module 200 from the optical instrument 300 in the housing and propagate to the outside of the housing.

Similarly, for the third to fifth modified examples illustrated in FIGS. 15 to 17, in which the arrangement of the piezoelectric element layer 230 is varied, the fact that light can pass through the ultrasound probe module 200 from the optical instrument 300 in the housing and propagate to the outside of the housing is the same.

Referring to FIG. 15, the piezoelectric elements constituting the piezoelectric element layer 230 may be arranged in a shape that forms a two-dimensional plane (2D Matrix) horizontally and vertically.

In this case, the stacked structure of the ultrasound probe module 200 formed in one direction as described above may be formed in two-dimensional directions, horizontally and vertically.

This case is advantageous because the ultrasound probe module 200 can easily obtain a three-dimensional image.

Referring to FIG. 16, the piezoelectric elements constituting the piezoelectric element layer 230 may be formed in the shape of rings with different radii and arranged in an annular shape.

In this case, the lens 210, the matching layer 220, and the block layer 240 may also be formed in a ring shape according to the piezoelectric elements formed in the shape of rings.

Referring to FIG. 17, the piezoelectric elements constituting the piezoelectric element layer 230 may be arranged in a shape forming a hemisphere.

In this case, the stacked structure of the ultrasound probe module 200 formed in one direction as described above may be formed along the arrangement of each piezoelectric element.

This case is advantageous because the ultrasound probe module 200 can emit a signal by focusing the signal on a relatively narrower point than the modified example illustrated in FIG. 14.

By adopting the above structures, the optical apparatus 100 according to one embodiment of the present invention can improve a signal-to-noise ratio and reduce the size of a device, unlike the conventional optical apparatus in which the ultrasound probe and the optical instrument are coupled non-coaxially.

In addition, the optical apparatus 100 according to one embodiment of the present invention can obtain matching ultrasound image information and optical image information throughout the entire area L3 in the depth direction of the specimen 500.

Accordingly, images having various types of information can be simultaneously obtained, thereby increasing the accuracy of lesion diagnosis.

At the same time, the optical apparatus 100 according to one embodiment of the present invention may use characteristics such as light absorption and light scattering to generate imaging information by targeting a specific tissue through fluorescent labeling, or perform broader tasks such as measuring oxygen saturation using a light absorption degree.

Further, the optical apparatus 100 according to one aspect of the present invention can improve image quality and provide a wide viewing angle by applying multiple piezoelectric elements.

Further, the optical apparatus 100 according another aspect of the present invention allows the arrangement of multiple piezoelectric elements to be diversified, so that the optical apparatus can be provided according to the purpose.

With the above configuration, an optical apparatus according to one aspect of the present invention can improve a signal-to-noise ratio (SNR) and reduce a size of a device by adopting a configuration in which a transparent ultrasound probe module is disposed coaxially with the optical apparatus.

Further, an optical apparatus according to another aspect of the present invention can provide an ultrasound probe module having high transparency by using a transparent material such as lithium niobate.

Further, an optical apparatus according to still another aspect of the present invention can improve image quality and provide a wide viewing angle by applying multiple piezoelectric elements.

Further, an optical apparatus according to yet another aspect of the present invention allows the arrangement of multiple piezoelectric elements to be diversified, so that the optical apparatus can be provided according to the purpose.

Effects of the present invention are not limited to the above-mentioned effects, and it should be understood that the present invention includes all effects inferable from the detailed description of the present invention or the constitution of the invention described in the claims.

The embodiments of the present invention have been described above. However, it should be noted that the spirit of the present invention is not limited to the embodiments in the specification and those skilled in the art and understanding the present invention may easily suggest other embodiments by addition, modification, and removal of the components within the same spirit, and those are construed as being included in the spirit of the present invention.

## Claims

1. A transparent ultrasound probe module provided inside a housing of an optical apparatus, the transparent ultrasound probe module comprising:
a lens provided on one side of an inside of the housing, formed of a transparent material, and disposed such that one surface thereof is exposed to the outside of the housing;
a first matching layer provided on the other surface of the lens, and formed of a transparent material;
a piezoelectric element layer provided on the first matching layer and including a plurality of transparent piezoelectric elements capable of interconverting an electrical signal and a mechanical signal;
a first electrode layer and a second electrode layer formed of a transparent material, provided on one surface and the other surface of the piezoelectric element layer, respectively, and configured to transmit an electrical signal;
circuit boards connected to each of the first electrode layer and the second electrode layer, formed of a bendable material, and configured to transmit and receive the electrical signal to and from the piezoelectric element; and
a block layer provided on the second electrode layer, formed of a transparent material, and configured to remove ultrasound noise.

2. The transparent ultrasound probe module of claim 1, further comprising a second matching layer formed of a transparent material, between the first matching layer and the lens.

3. The transparent ultrasound probe module of claim 1, wherein the circuit boards are formed of a transparent material.

4. The transparent ultrasound probe module of claim 1, wherein the circuit boards include a ground board (ground fpcb) or a gold foil plate performing a grounding function and a signal board (signal fpcb) for signal transmission.

5. The transparent ultrasound probe module of claim 4, wherein
the ground fpcb or the gold foil plate is connected to the first electrode layer, between the first electrode layer and the first matching layer, and
the signal fpcb is connected to the second electrode layer, between the second electrode layer and the block layer.

6. The transparent ultrasound probe module of claim 5, wherein
the block layer is formed to have a wider surface than each of the piezoelectric element layer and the second electrode layer, and
the signal fpcb is disposed on the piezoelectric element layer, which is located on an upper surface of the block layer, and on a portion of the upper surface of the block layer protruding from the second electrode layer, and is connected to the second electrode layer through a conductive material.

7. The transparent ultrasound probe module of claim 1, wherein the plurality of piezoelectric elements constituting the piezoelectric element layer are disposed in a row.

8. The transparent ultrasound probe module of claim 7, wherein the plurality of piezoelectric elements are disposed in a row, and arranged in the shape of an arch protruding convexly upward.

9. The transparent ultrasound probe module of claim 7, wherein the plurality of piezoelectric elements are disposed in a row, and arranged in a U-shape that is concave upward.

10. The transparent ultrasound probe module of claim 1, wherein the plurality of piezoelectric elements constituting the piezoelectric element layer are disposed in a grid shape horizontally and vertically.

11. The transparent ultrasound probe module of claim 1, wherein the plurality of piezoelectric elements constituting the piezoelectric element layer are disposed in a circular shape.

12. The transparent ultrasound probe module of claim 11, wherein the plurality of piezoelectric elements are formed in the form of rings with different diameters and disposed in an annular shape.

13. The transparent ultrasound probe module of claim 11, wherein the plurality of piezoelectric elements are disposed in a hemispherical shape having a constant curvature.

14. An optical/ultrasound optical apparatus comprising:
a housing;
the transparent ultrasound probe module of any one of claims 1 to 17 provided on one side of an inside of the housing; and
an optical instrument provided on the other side of the inside of the housing and disposed coaxially with the transparent ultrasound probe module in a longitudinal direction of the housing.

15. The transparent ultrasound probe module of claim 14, further comprising an optical lens provided inside the housing, and disposed between the optical instrument and the transparent ultrasound probe module.
